# EUROPÄISCHE PATENTSCHRIFT

(11) **EP 1 183 227 B1**
(45) Veröffentlichungstag und Bekanntmachung des Hinweises auf die Patenterteilung: **04.02.2004**
(21) Anmeldenummer: 01919309.3
(22) Anmeldetag: 20.02.2001
(51) Int. Cl.: C07C 211/53, C07C 215/68, C07C 215/16, A61K 7/13

(54) **P-DIAMINOBENZOL-DERIVATE UND DIESE VERBINDUNGEN ENTHALTENDE FÄRBEMITTEL**
P-DIAMINOBENZENE DERIVATIVES AND DYES CONTAINING SAID COMPOUNDS
DERIVES DE P-DIAMINOBENZOL ET COLORANTS CONTENANT CES COMPOSES

(30) Priorität: 24.03.2000 DE 10014855
(43) Veröffentlichungstag der Anmeldung: 06.03.2002
(73) Patentinhaber: Wella Aktiengesellschaft, 64274 Darmstadt (DE)
(72) Erfinder: CHASSOT, Laurent, CH-1724 Praroman (CH)
(86) Internationale Anmeldenummer: PCT/EP2001/001860
(87) Internationale Veröffentlichungsnummer: WO 2001/072686

(56) Entgegenhaltungen:
- EP-A- 0 286 896
- DE-A- 19 822 041

## Beschreibung

Die Erfindung betrifft neue p-Diaminobenzol-Derivate sowie diese Verbindungen enthaltende Mittel zum Färben von Keratinfasern.

Auf dem Gebiet der Färbung von Keratinfasem, insbesondere der Haarfärbung, haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Als Entwicklersubstanzen werden hierbei insbesondere 2,5-Diaminotoluol, 2,5-Diaminophenylethylalkohol, p-Aminophenol, 4-Amino-3-methyl-phenol und 1,4-Diaminobenzol eingesetzt, während als Kupplersubstanzen beispielsweise Resorcin, 4-Chlorresorcin, 1-Naphthol, 3-Aminophenol, 5-Amino-2-methylphenol und Derivate des m-Phenylendiamins zu nennen sind. Weiterhin werden in der DE-OS 198 22 041 Färbemittel beschrieben, welche als Entwicklersubstanzen in 2-Stellung substituierte p-Phenylendiamine enthalten.

An Oxidationsfarbstoffe, die zur Färbung menschlicher Haare verwendet werden, werden neben der Färbung in der gewünschten Intensität zahlreiche zusätzliche Anforderungen gestellt. So müssen die Farbstoffe in toxikologischer und dermatologischer Hinsicht unbedenklich sein und die erzielten Haarfärbungen eine gute Lichtechtheit, Dauerwellechtheit, Säureechtheit und Reibeechtheit aufweisen. Auf jeden Fall aber müssen solche Färbungen ohne Einwirkung von Licht, Reibung und chemischen Mitteln über einen Zeitraum von mindestens 4 bis 6 Wochen stabil bleiben. Außerdem ist es erforderlich, daß durch Kombination geeigneter Entwicklersubstanzen und Kupplersubstanzen eine breite Palette verschiedener Farbnuancen erzeugt werden kann.

Mit den derzeit eingesetzten Färbemitteln ist es jedoch nicht möglich, die vorgenannten Anforderungen in allen Punkten zu erfüllen.

Es bestand daher weiterhin ein Bedürfnis nach neuen Entwicklersubstanzen, welche die vorgenannten Anforderung in besonderem Masse erfüllen.

Hierzu wurde nun überraschenderweise gefunden, dass die p-Diaminobenzol-Derivate der allgemeinen Formel (I) die an Entwicklersubstanzen gestellten Anforderungen in besonders hohem Masse erfüllen. So werden unter Verwendung dieser Entwicklersubstanzen mit bekannten Kupplersubstanzen farbstarke Farbnuancen erhalten, die außerordentlich lichtecht und waschecht sind.

Gegenstand der vorliegenden Erfindung sind daher p-Diaminobenzol-Derivate der allgemeinen Formel (I) oder deren physiologisch verträgliche, wasserlösliche Salze, 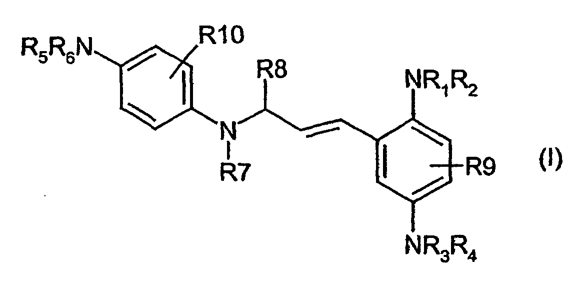 worin
- **R1, R2, R3, R4, R5,R6 und R7**: unabhängig voneinander Wasserstoff, eine C₁-C₆-Alkylgruppe, eine C₁-C₄-Hydroxyalkylgruppe, eine C₂-C₄-Dihydroxyalkylgruppe oder eine C₁-C₄-Alkoxy-(C₁-C₄)alkylgruppe darstellen, oder **R1**und **R2** beziehungsweise **R3** und **R4** beziehungsweise **R5** und **R6** einen viergliedrigen bis achtglidrieger aliphatischen Ring bilden, wobei mindestens zwei der Reste **R1** bis **R7** Wasserstoff bedeuten;
- **R8**: gleich Wasserstoff oder einer C₁-C₄-Alkylgruppe ist;
- **R9, R10**: unabhängig voneinander gleich Wasserstoff, einer Hydroxygruppe, einem Halogenatom, einer C₁-C₄-Alkylgruppe, einer C₁-C₄-Hydroxyalkylgruppe, einer C₁-C₄-Aminoalkylgruppe, einer Aminogruppe, einer C₁-C₂-Alkylaminogruppe oder einer C₁-C₄-Alkoxy-gruppe ist.

Als Verbindungen der Formel (I) können beispielweise genannt werden: 4-[3-(2,5-Diamino-phenyl)-allylamino]-anilin; 4-[3-(2,5-Diamino-phenyl)-allylamino)-2-(2-hydroacyethyl)-anilin; 4-[3-(2,5-Diamino-phenyl)-allylamino)-5-(2-hydroxyethyl)-anilin; 4-[3-(2,5-Diamino-phenyl)-allylamino)-2-chlor-anilin; 4-[3-(2,5-Diamino-phenyl)-allylamino)-2-methyl-anilin; 4-[3-(2,5-Diamino-phenyl)-allylamino)-3-(2-hydroxyethyl)-anilin; 4-[3-(2,5-Diamino-phenyl)-allylamino)-3-chlor-anilin; 4-[3-(2,5-Diamino-phenyl)-allylamino)-3-methyl-anilin; 4-[3-(2,5-Diamino-3-(2-hydroxyethyl)-phenyl)-allylamino)-anilin; 4-[3-(2,5-Diamino-3-chlor-phenyl)-allylamino)-anilin; 4-[3-(2,5-Diamino-3-methyl-phenyl)-allylamino)-anilin; 4-[3-(2,5-Diamino-6-methyl-phenyl)-allylamino)-anilin; 4-[3-(2,5-Diamino-4-(2-hydroxyethyl)-phenyl)-allylamino)-anilin; 4-[3-(2,5-Diamino-4-chlor-phenyl)-allylamino)-anilin; 4-[3-(2,5-Diamino-4-methyl-phenyl)-allylamino)-anilin; 4-[3-(N²,N²-Bis-methyl-2,5-diamino-phenyl)-allylamino)-2-anilin; 4-[3-(N²,N²-Bis-(2-hydroxyethyl)-2,5-diamino-phenyl)-allylamino)-2-anilin; 4-[3-(N⁵,N⁵-Bis-methyl-2,5-diamino-phenyl)-allylamino)-2-anilin; 4-[3-(N⁵,N⁵-Bis-(2-hydroxyethyl)-2,5-diamino-phenyl)-allylamino)-2-anilin, 4-[3-(2,5-Diamino-phenyl)-allylamino)-2-N,N'-bis-methyl-anilin; 4-[3-(2,5-Diamino-phenyl)-allylamino)-2-N,N'-bis-(2-hydroxyethyl)-anilin oder deren physiologisch verträglichen Salze.

Bevorzugt sind Verbindungen der Formel (I), in denen (i) mindestens einer der Reste **R8** bis **R10** Wasserstoff bedeutet und/oder (ii) **R1** und **R2** oder **R3** und **R4** oder **R5** und **R6** oder alle Reste **R1** bis **R4** beziehungsweise **R1** bis **R7** Wasserstoff bedeuten. Bevorzugt sind weiterhin Verbindungen der Formel (I), in denen **R8** gleich Wasserstoff und **R5** und **R6** unabhängig voneinander gleich Wasserstoff, einer C₁-C₄-Alkylgruppe, einer C₁-C₄-Hydroxyalkylgruppe, einer C₂-C₄-Dihydroxyalkylgruppe sind.

Besonders bevorzugte p-Diaminobenzol-Derivate der Formel (I) sind hierbei 4-[3-(2,5-Diamino-phenyl)-allylamino]-anilin; 4-[3-(2,5-Diamino-phenyl)-allylamino)-2-(2-hydroxyethyl)-anilin; 4-[3-(2,5-Diamino-phenyl)-allylamino]-2-methyl-anilin; 4-[3-(2,5-Diamino-phenyl)-allylamino]-3-methyl-anilin; 4-[3-(2,5-Diamino-phenyl)-allylamino)-3-(2-hydroxyethyl)-anilin; 4-[3-(2,5-Diamino-phenyl)-allylamino)-2-N,N'-bis-methyl-anilin; 4-[3-(2,5-Diamino-phenyl)-allylamino)-2-N,N'-bis-(2-hydroxyethyl)-anilin oder deren physiologisch verträgliche Salze.

Die Verbindungen der Formel (I) können sowohl als freie Basen als auch in Form ihrer physiologisch verträglichen Salze mit anorganischen oder organischen Säuren, wie zum Beispiel Salzsäure, Schwefelsäure, Phosphorsäure, Essigsäure, Propionsäure, Milchsäure oder Zitronensäure, eingesetzt werden.

Die Herstellung der erfindungsgemäßen p-Diaminobenzol-Derivate der Formel (I) kann unter Verwendung von bekannten Syntheseverfahren erfolgen. Die Synthese der erfindungsgemäßen Verbindungen kann beispielsweise wie folgt durchgeführt werden:
Durch eine reduktive Aminierung eines substituierten Benzols der Formel (II) 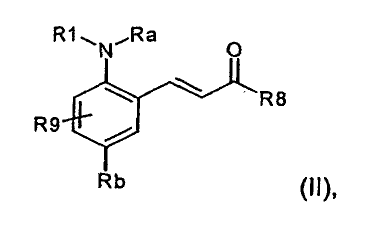 worin Ra für eine geeignete Schutzgruppe, wie sie zum Beispiel in Organic Synthesis, Kapitel 7, "Protection for the Amino Group," Seite 309 ff., Wiley Interscience, 1991 beschrieben wird, steht; Rb die Bedeutung NR1Ra oder NR1R2 hat, mit einem Amin der Formel (III) 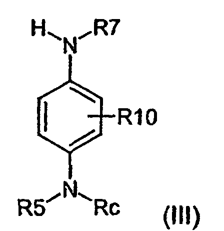 worin Rc die Bedeutung Ra oder R6 hat,
wobei R5, R6, R7, R8, R9 und R10 die in Formel (I) genannte Bedeutung haben, und anschliessende Abspaltung der Schutzgruppe.

Die erfindungsgemäßen p-Diaminobenzol-Derivate der Formel (I) sind in Wasser gut löslich und ermöglichen Färbungen mit hoher Farbintensität und ausgezeichneter Farbechtheit, insbesondere was die Lichtechtheit, Waschechtheit und Reibeechtheit anbetrifft. Die Verbindungen der Formen (I) weisen weiterhin eine ausgezeichnete Lagerstabilität, insbesondere als Bestandteil der nachfolgend beschriebenen Färbemittel, auf.

Ein weiterer Gegenstand der vorliegenden Erfindung sind daher Mittel zum oxidativen Färben von Keratinfasern, wie zum Beispiel Haaren, Pelzen, Federn oder Wolle, insbesondere menschlichen Haaren, auf der Basis einer Entwicklersubstanz-Kupplersubstanz-Kombination, welche als Entwicklersubstanz mindestens ein p-Diaminobenzol-Derivat der Formel (I) enthalten.

Die p-Diaminobenzol-Derivate der Formel (I) sind in dem erfindungsgemässen Färbemittel in einer Gesamtmenge von etwa 0,005 bis 20 Gewichtsprozent enthalten, wobei eine Menge von etwa 0,01 bis 5,0 Gewichtsprozent und insbesondere von 0,1 bis 5 Gewichtsprozent bevorzugt ist.

Als Kupplersubstanzen kommen vorzugsweise 2,6-Diamino-pyridin, 2-Amino-4-[(2-hydroxyethyl)amino]-anisol, 2,4-Diamino-1-fluor-5-methylbenzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydrox-phenyl)amino]-acetamid, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-[(2-Hydroxyethyl)amino]-2-methyl-phenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphtholacetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol,7-Hydroxy-indol und 2,3-Indolindion in Betracht.

Obwohl die vorteilhaften Eigenschaften der hier beschriebenen p-Diaminobenzol-Derivate der Formel (I) es nahelegen, diese als alleinige Entwicklersubstanz zu verwenden, ist es selbstverständlich auch möglich, die p-Diaminobenzol-Derivate der Formel (I) gemeinsam mit bekannten Entwicklersubstanzen, wie zum Beispiel 1,4-Diaminobenzol, 2,5-Diaminotoluol, 2,5-Diaminophenylethylalkohol, 4-Aminophenol und seinen Derivaten, beispielsweise 4-Amino-3-methylphenol, 4,5-Diaminopyrazolderivaten, zum Beispiel 4,5-Diamino-1-(2-hydroxyethyl)-pyrazol, oder Tetraaminopyrimidinen, einzusetzen.

Die Kupplersubstanzen und Entwicklersubstanzen können in dem erfindungsgemäßen Färbemittel jeweils einzeln oder im Gemisch miteinander enthalten sein, wobei die Gesamtmenge an Kupplersubstanzen und Entwicklersubstanzen in dem erfindungsgemässen Färbemittel (bezogen auf die Gesamtmenge des Färbemittels) jeweils etwa 0,005 bis 20 Gewichtsprozent, vorzugsweise etwa 0,01 bis 5,0 Gewichtsprozent und insbesondere 0,1 bis 2,5 Gewichtsprozent, beträgt.

Die Gesamtmenge der in dem hier beschriebenen Färbemittel enthaltenen Entwicklersubstanz-Kupplersubstanz-Kombination beträgt vorzugsweise etwa 0,01 bis 20 Gewichtsprozent, wobei eine Menge von etwa 0,02 bis 10 Gewichtsprozent und insbesondere 0,2 bis 6,0 Gewichtsprozent besonders bevorzugt ist. Die Entwicklersubstanzen und Kupplersubstanzen werden im allgemeinen in etwa äquimolaren Mengen eingesetzt; es ist jedoch nicht nachteilig, wenn die Entwicklersubstanzen diesbezüglich in einem gewissen Uberschuß oder Unterschuß vorhanden sind (beispielsweise in einem Verhältnis von Kupplersubstanz zu Entwicklersubstanz von 1:2 bis 1:0,5).

Weiterhin kann das erfindungsgemässe Färbemittel zusätzlich andere Farbkomponenten, beispielsweise 6-Amino-2-methylphenol und 2-Amino-5-methylphenol, sowie ferner übliche direktziehende Farbstoffe, zum Beispiel Triphenylmethanfarbstoffe wie 4-[(4'-aminophenyl)-(4'imino-2",5"-cyclohexadien-1"-yliden)-methyl]-2-methylaminobenzol-monohydrochlorid (C.I. 42 510) und 4-[(4'amino-3'-methyl-phenyl)-(4"-imino-3"-methyl-2",5"cyclohexadien-1"-yliden)-methyl]-2-methyl-aminobenzol monohydrochlorid (C.I. 42 520), aromatische Nitrofarbstoffe wie 4-(2'-hydroxyethyl)amino-nitrotoluol, 2-Amino-4,6-dinitrophenol, 2-Amino-5-(2'-hydroxyethyl)amino-nitrobenzol, 2-Chlor-6-(ethylamino)-4-nitrophenol, 4-Chlor-N-(2-hydroxyethyl)-2-nitroanilin, 5-Chlor-2-hydroxy-4-nitroanilin, 2-Amino-4-chlor-6-nitrophenol und 1-[(2'-Ureidoethyl)amino-4-nitrobenzol, Azofarbstoffe wie 6-[(4'-Aminophenyl)azo]-5hydroxynaphthalin-1-sulfonsäure-Natriumsalz (C.I. 14 805) und Dispersionsfarbstoffe wie beispielsweise 1,4-Diaminoanthrachinon und 1,4,5,8-Tetraamino-antrachinon, enthalten.
Die erfindungsgemäßen Färbemittel können diese Farbkomponenten in einer Menge von etwa 0,1 bis 4,0 Gewichtsprozent enthalten.

Selbstverständlich können die Kupplersubstanzen und Entwicklersubstanzen sowie die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Darüber hinaus können in den Färbemitteln, falls diese zur Färbung von Haaren verwendet werden sollen, noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein. Die Zubereitungsform des erfindungsgemässen Färbemittels kann beispielsweise eine Lösung, insbesondere eine wässrige oder wässrigalkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Ubliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie hohere Fettalkohole, Stärke, Cellulosederivate, Petrolatum, Paraffinöl und Fettsäuren, sowie ausserdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Je nach Zusammensetzung kann das erfindungsgemäße Färbemittel schwach sauer, neutral oder alkalisch reagieren. Vorzugsweise weist das erfindungsgemäße Färbemittel einen pH-Wert von 5 bis 11,5 auf, wobei ein pH-Wert von etwa 6,5 bis 10,5 besonders bevorzugt ist. Die basische Einstellung erfolgt vorzugsweise mit Ammoniak, es können aber auch organische Amine, zum Beispiel Monoethanolamin und Triethanolamin, oder auch anorganische Basen wie Natriumhydroxid und Kaliumhydroxid verwendet werden. Für eine pH-Einstellung im sauren Bereich kommen anorganische oder organische Säuren, zum Beispiel Phosphorsäure, Essigsäure Zitronensäure oder Weinsäure, in Betracht.

Für die Anwendung zur oxidativen Färbung von Haaren vermischt man das vorstehend beschriebene Färbemittel unmittelbar vor dem Gebrauch mit einem Oxidationsmittel und trägt eine für die Haarfärbebehandlung ausreichende Menge, je nach Haarfülle, im allgemeinen etwa 60 bis 200 Gramm, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin, Natriumborat oder Natriumcarbonat in Form einer 3-bis 12prozentigen, vorzugsweise 6prozentigen, wässrigen Lösung, aber auch Luftsauerstoff in Betracht. Wird eine 6prozentige Wasserstoffperoxid-Lösung als Oxidationsmittel verwendet, so beträgt das Gewichtsverhältnis zwischen Haarfärbemittel und Oxidationsmittel 5:1 bis 1:2, vorzugsweise jedoch 1:1. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluss an diese Spülung mit einem Shampoo gewaschen und eventuell mit einer schwachen organischen Säure, wie zum Beispiel Zitronensäure oder Weinsäure, nachgespült. Anschliessend wird das Haar getrocknet.

Die erfindungsgemässen Haarfärbemittel mit einem Gehalt an Diaminobenzol-Derivaten der Formel (I) als Entwicklersubstanz ermöglichen Haarfärbungen mit ausgezeichneter Farbechtheit, insbesondere was die Lichtechtheit, Waschechtheit und Reibeechtheit anbetrifft. Hinsichtlich der färberischen Eigenschaften bieten die erfindungsgemässen Haarfärbemittel je nach Art und Zusammensetzung der Farbkomponenten eine breite Palette verschiedener Farbnuancen, welche sich von blonden über braune, purpume, violette bis hin zu blauen und schwarzen Farbtönen erstreckt. Hierbei zeichnen sich die Farbtöne durch ihre besondere Farbintensität aus. Die sehr guten färberischen Eigenschaften der Haarfärbemittel gemäß der vorliegenden Anmeldung zeigen sich weiterhin darin, dass diese Mittel eine Anfärbung von ergrauten, chemisch nicht vorgeschädigten Haaren problemlos und mit guter Deckkraft ermöglichen.

Die nachfolgenden Beispiele sollen den Gegenstand der Erfindung näher erläutern, ohne ihn darauf zu beschränken.

### Beispiele

### I. Herstellungsbeispiele

### Beispiele 1: Synthese von 4-[3-(2,5-Diamino-phenyl)allylamino]-anilin-Derivaten der Formel (I) (Allgemeine Synthesevorschrift)

### A. Synthese von 2,5-Bis-tert.-butyloxycarbonylamino-brombenzol

15,65g (0,07 mol) Brom-p-phenylendiamin-Hydrochlorid und 32,7 g (0,15 mol) Di-tert.-butyl-dicarbonat werden in einer Mischung von 250 ml 2n Natriunhydroxid und 250 ml Trifluortoluol gelöst und auf 45°C erwärmt. Die Reaktionmischung wird 3 Tage lang gerührt. Schrittweise werden noch insgesamt 30 g (0,14 mol) Di-tert.-butyl-dicarbonat zugegeben. Anschliessend wird die organische Schicht abgetrennt und die wässrige Phase noch zweimal mit 100ml Dichlormethan extrahiert. Die vereinigten Extrakte weren eingedampft und der Rückstand in 200 ml Hexan aufgenommen. Der Niederschlag wird abfiltriert und mit 50 ml Hexan nachgewaschen.
Es werden 18,6 g (82 % der Theorie) 2,5-tert.-Butyloxycarbonylaminobrombenzol mit einem Schmelzpunkt von 130 °C erhalten.

### B. Synthese von N-(4-tert.Butyloxycarbonylamino-2-formyl-phenyl)-carbaminsäure-tert.butylester

3,3 g (0,01 mol) 2,5-tert.-Butyloxycarbonylamino-brombenzol aus Stufe A werden unter Argon in 100 ml wasserfreiem Tetrahydrofuran gelöst. Schrittweise werden sodann 17 ml einer 1,6 molaren etherischen Methyllithiumlösung (0,03 mol) zugegeben. Die Reaktionsmischung wird auf -20 °C gekühlt, und schrittweise mit 7 ml einer 1,5 molaren tert.-Butyllithiumlösung (0,01 mol) versetzt. Nach beendeter Zugabe wird die Lösung noch 30 Minuten lang bei der angegebenen Temperatur gerührt. Anschliessend werden 1,2 g Dimethylformamid (0,02 mol) zugegeben und die Reaktionmischung wird eine Stunde lang bei -20 °C gerührt. Nach langsamer Erwämung auf Raumtemperatur wird die Reaktion mit Wasser hydrolisiert und dann auf Diethylether gegossen. Die wässerige Phase wird sodann mit Diethylether extrahiert und die organische Phase mit Magnesiumsulfat getrocknet. Das Lösungsmittel wird am Rotationsverdampfer abdestilliert und der Rückstand an Kieselgel mit Petrolether/Essigsäureethylester (9:1) gereinigt.

### C. Synthese von N-(4-tert-Butoxycarbonylamino-3-(3-oxo-propenyl)-phenyl)-carbaminsäure-tert-butylester

9,5 g (0,03mol) von N-(4-tert.-Butyloxycarbonylamino-2-formyl-phenyl)-carbaminsäure-tert.-butylester aus Stufe B werden in 100 ml Tetrahydrofuran gelöst und wird mit 10,2 g (0,035 mol) Formylmethyltriphenylphosphoran versetzt. Die Reaktionmsichung wird 18 Stunde langbei 60 °C gerührt. Das Reaktionsgemisch wird anschliessend auf Wasser gegossen, mit Essigsäureethylester extrahiert und die organische Phase mit einer gesättigten wässrigen NaCl-Lösung gewaschen, über Natriumsulfat getrocknet und nach Filtration eingeengt. Die Flash-Chromatographie des Rohproduktes an Kieselgel mit Hexan/EtOAc ergab 4,84g (47 % der Theorie).
¹H-NMR (300 MHz, CDCl₃):
9,68 (d; J=7,6; 1 H); 7,66 (d; J=2.2; 1H); 7,57 (d; J=15,9; 1 H); 7,43 (d; J=8,6; 1H); 7,28 (dd; J¹=2,4; J²=8,7; 1 H); 6,75 (s; 1 H); 6,61 (dd; J¹=7,6; J²=15.9; 1H); 6,51 (s; 1H); 1,51 (s; 18H)

### D. Synthese von 4-[3-(2,5-Diamino-phenyl)allylamino]-anilinen

0,036 g (0,0001 mol) N-(4-tert-Butoxycarbonylamino-3-(3-oxo-propenyl)-phenyl)-carbaminsäure-tert-butylester aus Stufe C und 0,00015 mol des entsprechenden Amins werden in 1,2-Dichlorethan gelöst. Anschliessend werden 0,1 ml einer Essigsäurelösung (1 m in 1,2-Dichlorethan) und 0,06 g NaBH(OAc)₃ (0,0003 mol) zugegeben und die Reaktionmischung wird 5 bis 15 Stunden lang bei Raumtemperatur gerührt
Nach Beendigung der Reaktion wird die Reaktionsmischung in 10 ml Essigsäureethylester gegossen, die organische Phase mit gesättigter Natriumhydrogencarbonat-Lösung extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) gereinigt. Das so erhaltene Produkt wird in 4 ml Ethanol auf 50 °C erwärmt. Anschliessend werden zur Herstellung des Hydrochlorides 1,5 ml einer 2,9 molaren ethanolische Salzsäurelösung zugetropft. Der Niederschlag wird abfiltriert, zweimal mit 1 ml Ethanol gewaschen und sodann getrocknet.

### 1a. 4-[3-(2,5-Diamino-phenyl)-allylamino]-anilin Hydrochlorid

Verwendetes Amin: 4-tert.-Butyloxycarbonylamino-anilin
Ausbeute: 0,025 g ( 95 % der Theorie)
Masspektrum: M⁺ 254 (20)

### 1b. 4-[3-(2,5-Diamino-phenyl)-allylamino]-3-methyl-anilin Hydrochlorid und 4-[3-(2,5-Diamino-phenyl)-allylamino]-2-methyl-anilin Hydrochlorid

Verwendetes Amin: 4-tert.-Butyloxycarbonylamino-3-methyl-anilin beziehungsweise 4-tert.-Butyloxycarbonylamino-2-methyl-anilin Ausbeute: 0,025 g (93 % der Theorie)
Masspektrum: M⁺268 (20)

### 1c. 2-{5-Amino-2-[3-(2,5-diamino-phenyl)-allylamino]-phenyl}-ethanol Hvdrochlorid und 2-{2-Amino-5-[3-(2,5-diamino-phenyl)-allylamino]-phenyl}-ethanol Hydrochlorid

Verwendetes Amin: 4-tert.-Butyloxycarbonylamino-3-(2-hydroxyethyl)-anilin beziehungsweise 4-tert.-Butyloxycarbonylamino-2-(2-hydroxyethyl)-anilin
Ausbeute: 0,025 g (56 % der Theorie)
Masspektrum: MH⁺299 (100)

### Beispiele 2 bis 4: Haarfärbemittel

Es werden Haarfärbelösungen der folgenden Zusammensetzung hergestellt:

| | |
|---|---|
| 1,25 mmol | Entwicklersubstanz der Formel (I) gemäss Tabelle 1 |
| 1,25 mmol | Kupplersubstanz gemäß Tabelle 1 |
| 1,0 g | Kaliumoleat (8prozentige wässrige Lösung) |
| 1,0 g | Ammoniak (22prozentige wässrige Lösung) |
| 1,0 g | Ethanol |
| 0,3 g | Ascorbinsäure |
| ad 100,0 g | Wasser |

50 g der vorstehenden Färbelösung wird unmittelbar vor der Anwendung mit 50 g einer 6prozentigen wässrigen Wasserstoffperoxidlösung vermischt. Anschliessend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die resultierenden Färbungen sind in Tabelle 1 zusammengefaßt.

**Tabelle 1:**

| **Beispiel** | **Entwicklersubstanz der Formel (I)** | **Kupplersubstanz** | | | |
|---|---|---|---|---|---|
| | | **I.** **1,3-Di-hydroxy-benzol** | **II.** **1,3-Diamino-4-(2-hydroxy-ethoxy)-benzol-sulfat** | **III.** **5-Amino-2-methyl-phenol** | **IV.** **1-Naphtol** |
| **2.** | gemäß Beispiel **1a** | dunkelblond | blau | purpur | violett |
| **3.** | gemäß Beispiel **1b** | mittelblond | blau | purpur | grauviolett |
| **4.** | gemäß Beispiel **1c** | helllichtblond | blau | purpur | violett |

### Beispiele 5 bis 14: Haarfärbemittel

Es werden Haarfärbelösungen der folgenden Zusammensetzung hergestellt:

| | |
|---|---|
| X g | 4-[3-(2,5-Diamino-phenyl)-allylamino]-anilin Hydrochlorid (Entwicklersubstanz **E1** der Formel (I)) |
| U g | Entwicklersubstanz **E2** bis **E9** gemäß Tabelle 2 |
| Y g | Kupplersubstanz **K11** bis **K36** gemäß Tabelle 4 |
| Z g | direktziehender Farbstoff **D1** bis **D3** gemäß Tabelle 3 |
| 10,000 g | Kaliumoleat (8prozentige wässrige Lösung) |
| 10,000 g | Ammoniak (22prozentige wässrige Lösung) |
| 10,000 g | Ethanol |
| 0,300 g | Ascorbinsäure |
| ad 100,000 g | Wasser |

30 g der vorstehenden Färbelösung werden unmittelbar vor der Anwendung mit 30 g einer 6prozentigen wässsrigen Wasserstoffperoxidlösung vermischt. Anschliessend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Die Färbeergebnisse sind in Tabelle 5 zusammengefasst.

### Beispiele 15 bis 20: Haarfärbemittel

Es werden cremeförmige Farbträgermassen der folgenden Zusammensetzung hergestellt:

| | |
|---|---|
| X g | 4-[3-(2,5-Diamino-phenyl)-allylamino]-anilin Hydrochlorid (Entwicklersubstanz **E1** der Formel (I)) |
| Y g | Kupplersubstanz **K11** bis **K36** gemäss Tabelle 4 |
| Z g | direktziehender Farbstoff **D2** gemäss Tabelle 3 |
| 15,0 g | Cetylalkohol |
| 0,3 g | Ascorbinsäure |
| 3,5 g | Natriumlaurylalkoholdiglycolethersulfat, 28%ige wässrige Lösung |
| 3,0 g | Ammoniak 22%ige wässrige Lösung |
| 0,3 g | Natriumsulfit, wasserfrei |
| ad 100 g | Wasser |

30 g der vorstehenden Färbecreme werden unmittelbar vor der Anwendung mit 30 g einer 6prozentigen Wasserstoffperoxidlösung vermischt. Anschliessend wird das Gemisch auf das Haar aufgetragen. Nach einer Einwirkzeit von 30 Minuten wird das Haar mit Wasser gespült, mit einem handelsüblichen Shampoo gewaschen und getrocknet. Die Färbeergebnisse sind in Tabelle 6 zusammengefasst.

**Tabelle 2:**

| **Entwicklersubstanzen** | |
|---|---|
| **E2** | 1,4-Diaminobenzol |
| **E3** | 2,5-Diamino-phenylethanol-sulfat |
| **E4** | 3-Methyl-4-amino-phenol |
| **E5** | 4-Amino-2-aminomethyl-phenol-dihydrochlorid |
| **E6** | 4-Amino-phenol |
| **E7** | N,N-Bis(2'-hydroxyethyl)-p-phenylendiamin-sulfat |
| **E8** | 4,5-Diamino-1-(2'-hydroxyethyl)-pyrazol-sulfat |
| **E9** | 2,5-Diaminotoluol-sulfat |

**Tabelle 3:**

| **Direktziehende Farbstoffe** | |
|---|---|
| **D1** | 2,6-Diamino-3-((pyridin-3-yl)azo)pyridin |
| **D2** | 6-Chlor-2-ethylamino-4-nitro-phenol |
| **D3** | 2-Amino-6-chlor-4-nitro-phenol |

**Tabelle 4:**

| **Kupplersubstanzen** | |
|---|---|
| **K11** | 1,3-Diaminobenzol |
| **K12** | 2-Amino-4-(2'-hydroxyethyl)amino-anisol-sulfat |
| **K13** | 1,3-Diamino-4-(2'-hydroxyethoxy)benzol-sulfat |
| **K14** | 2,4-Diamino-5-fluor-toluol-sulfat |
| **K15** | 3-Amino-2-methylamino-6-methoxy-pyridin |
| **K16** | 3,5-Diamino-2,6-dimethoxy-pyridin-dihydrochlorid |
| **K17** | 2,4-Diamino-5-ethoxy-toluol-sutfat |
| **K18** | N-(3-Dimethylamino)phenylhamstoff |
| **K19** | 1,3-Bis(2,4-Diaminophenoxy)propan-tetrahydrochlorid |
| **K21** | 3-Amino-phenol |
| **K22** | 5-Amino-2-methyl-phenol |
| **K23** | 3-Amino-2-chlor-6-methyl-phenol |
| **K24** | 5-Amino-4-fluor-2-methyl-phenol-sulfat |
| **K25** | 1-Naphthol |
| **K26** | 1-Acetoxy-2-methyl-naphthalin |
| **K31** | 1,3-Dihydroxy-benzol |
| **K32** | 2-Methyl-1,3-dihydroxy-benzol |
| **K33** | 1-Chlor-2,4-dihydroxy-benzol |
| **K34** | 4-(2'-Hydroxyethyl)amino-1,2-methylendioxybenzol-hydrochlorid |
| **K35** | 3,4-Methylendioxy-phenol |
| **K36** | 2-Amino-5-methyl-phenol |

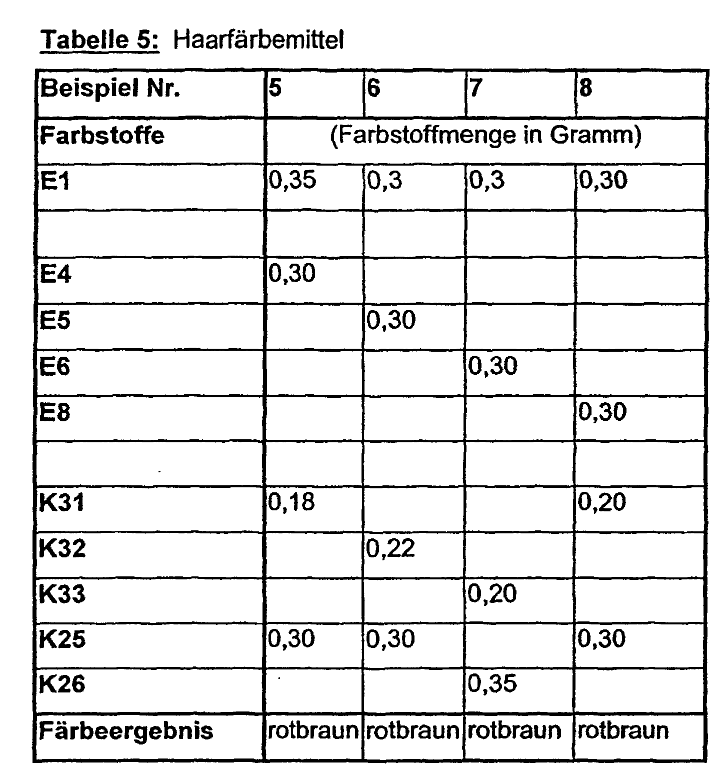

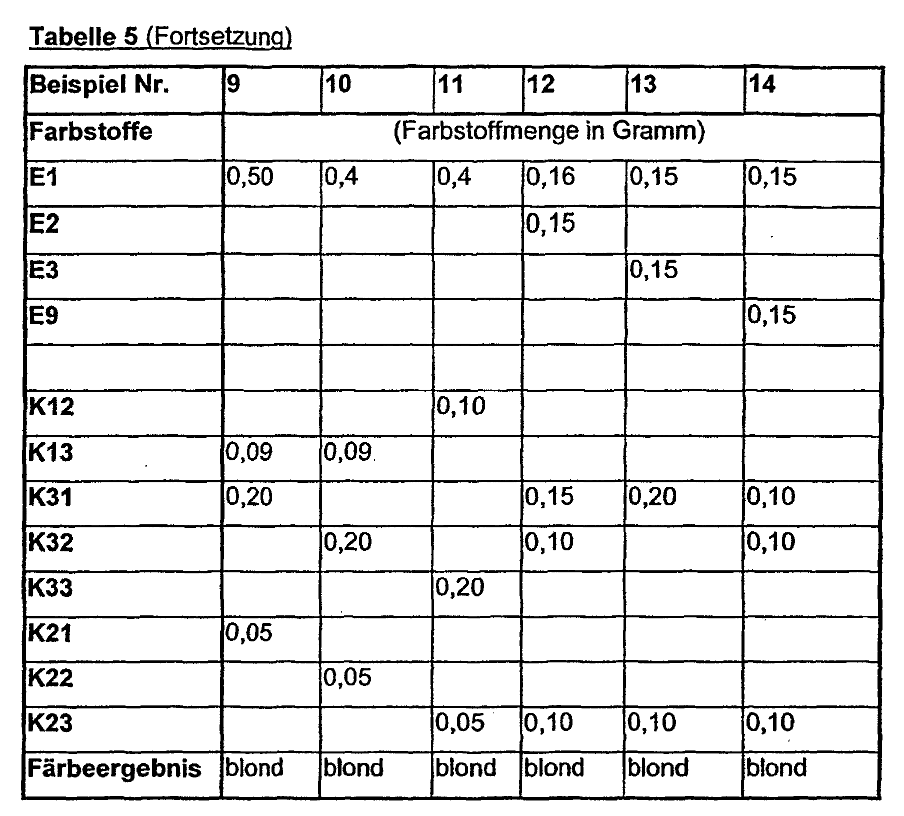

**Tabelle 6:**

| Haarfärbemittel | | | | | | |
|---|---|---|---|---|---|---|
| **Beispiel Nr.** | **15** | **16** | **17** | **18** | **19** | **20** |
| **Farbstoffe** | (Farbstoffmenge in Gramm) | | | | | |
| **E1** | 2,50 | 2,50 | 2,50 | 0,90 | 0,90 | 0,90 |
| | | | | | | |
| **K12** | | | | 0,10 | 0,10 | 0,10 |
| **K13** | 1,10 | 1,10 | 1,10 | | | |
| **K31** | 1,10 | 1,10 | 1,10 | 0,40 | 0,40 | 0,40 |
| | | | | | | |
| **D2** | | | | 0,10 | 0,10 | 0,10 |
| **K23** | | | 0,05 | 0,10 | 0,10 | 0,10 |
| **Färbeergebnis** | schwarz | schwarz | schwarz | braun | braun | braun |

Alle in der vorliegenden Anmeldung enthaltenen Prozentangaben stellen soweit nicht anders angegeben Gewichtsprozente dar.

## Patentansprüche

1. p-Diaminobenzol-Derivate der allgemeinen Formel (I) oder deren physiologisch verträgliche, wasserlösliche Salze, 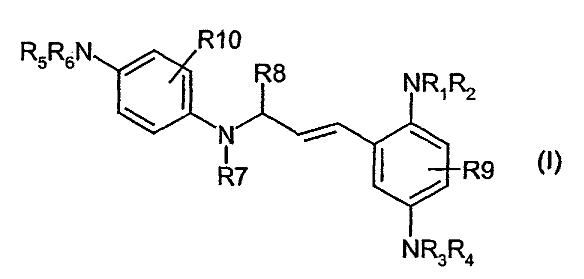 worin
**R1, R2, R3, R4, R5,R6 und R7** unabhängig voneinander Wasserstoff, eine C₁-C₆-Alkylgruppe, eine C₁-C₄-Hydroxyalkylgruppe, eine C₂-C₄-Dihydroxyalkylgruppe oder eine C₁-C₄-Alkoxy-(C₁-C₄)alkylgruppe darstellen, oder **R1**und **R2** beziehungsweise **R3** und **R4** beziehungsweise **R5** und **R6** einen viergliedrigen bis achtglidrieger aliphatischen Ring bilden, wobei mindestens zwei der Reste **R1** bis **R7** Wasserstoff bedeuten;
**R8** gleich Wasserstoff oder einer C₁-C₄-Alkylgruppe ist;
**R9, R10** unabhängig voneinander gleich Wasserstoff, einer Hydroxygruppe, einem Halogenatom, einer C₁-C₄-Alkylgruppe, einer C₁-C₄-Hydroxyalkylgruppe, einer C₁-C₄-Aminoalkylgruppe, einer Aminogruppe, einer C₁-C₂-Alkylaminogruppe oder einer C₁-C₄-Alkoxygruppe ist.

2. p-Diaminobenzolderivat gemäß Anspruch 1, **dadurch gekennzeichnet, dass** in der Formel (I) (i) mindestens einer der Reste **R8** bis **R10** Wasserstoff bedeutet und/oder (ii) **R1** und **R2** oder **R3** und **R4** oder **R5** und **R6** oder alle Reste **R1** bis **R4** beziehungsweise **R1** bis **R7** Wasserstoff bedeuten.

3. p-Diaminobenzolderivat gemäß Anspruch 1 oder 2, **dadurch gekennzeichnet, dass** in der Formel (I) **R8** gleich Wasserstoff und **R5** und R6 unabhängig voneinander gleich Wasserstoff, einer C₁-C₄-Alkylgruppe, einer C₁-C₄-Hydroxyalkylgruppe, einer C₂-C₄-Dihydroxyalkylgruppe sind.

4. p-Diaminobenzolderivat gemäß einem der Ansprüche 1 bis 3, **dadurch gekennzeichnet, dass** es ausgewählt ist aus der Gruppe bestehend aus 4-[3-(2,5-Diamino-phenyl)-allylamino]-anilin; 4-[3-(2,5-Diamino-phenyl)-allylamino)-2-(2-hydroxyethyl)-anilin; 4-[3-(2,5-Diamino-phenyl)-allylamino)-5-(2-hydroxyethyl)-anilin; 4-[3-(2,5-Diamino-phenyl)-allylamino)-2-chlor-anilin; 4-[3-(2,5-Diamino-phenyl)-allylamino)-2-methyl-anilin; 4-[3-(2,5-Diamino-phenyl)-allylamino)-3-(2-hydroxyethyl)-anilin; 4-[3-(2,5-Diamino-phenyl)-allylamino)-3-chlor-anilin; 4-[3-(2,5-Diamino-phenyl)-allylamino)-3-methyl-anilin; 4-[3-(2,5-Diamino-3-(2-hydroxyethyl)-phenyl)-allylamino)-anilin; 4-[3-(2,5-Diamino-3-chlor-phenyl)-allylamino)-anilin; 4-[3-(2,5-Diamino-3-methyl-phenyl)-allylamino)-anilin; 4-[3-(2,5-Diamino-6-methyl-phenyl)-allylamino)-anilin; 4-[3-(2,5-Diamino-4-(2-hydroxyethyl)-phenyl)-allylamino)-anilin; 4-[3-(2,5-Diamino-4-chlor-phenyl)-allylamino)-anilin; 4-[3-(2,5-Diamino-4-methyl-phenyl)-allylamino)-anilin; 4-[3-(N²,N²-Bis-methyl-2,5-diamino-phenyl)-allylamino)-2-anilin; 4-[3-(N²,N²-Bis-(2-hydroxyethyl)-2,5-diamino-phenyl)-allylamino)-2-anilin; 4-[3-(N⁵,N⁵-Bis-methyl-2,5-diamino-phenyl)-allylamino)-2-anilin; 4-[3-(N⁵,N⁵-Bis-(2-hydroxyethyl)-2,5-diamino-phenyl)-allylamino)-2-anilin, 4-[3-(2,5-Diamino-phenyl)-allylamino)-2-N,N'-bis-methyl-anilin; 4-[3-(2,5-Diamino-phenyl)-allylamino)-2-N,N'-bis-(2-hydroxyethyl)-anilin oder deren physiologisch verträglichen Salze.

5. Mittel zum oxidativen Färben von Keratinfasern auf der Basis einer Entwicklersubstanz-Kupplersubstanz-Kombination, **dadurch gekennzeichnet, dass** es als Entwicklersubstanz mindestens ein p-Diaminobenzolderivat der Formel (I) gemäß einem der Ansprüche 1 bis 4 enthält.

6. Mittel nach Anspruch 5, **dadurch gekennzeichnet, dass** es das Diaminobenzolderivat der Formel (I) in einer Menge von 0,005 bis 20 Gewichtsprozent enthält.

7. Mittel nach Anspruch 5 oder 6, **dadurch gekennzeichnet, dass** die Kupplersubstanz ausgewählt ist aus der Gruppe bestehend aus 2,6-Diamino-pyridin, 2-Amino-4-[(2-hydroxyethyl)amino]-anisol, 2,4-Diamino-1-fluor 5-methyl-benzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol; 2-[(3-Hydrox-phenyl)amino]-acetamid, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxy-propyl)-amino]-2-methyl-phenol, 3-[(2-Hydroxyethyl)amino)-2-methyl-phenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion.

8. Mittel einem der Ansprüche 5 bis 7, **dadurch gekennzeichnet, dass** es ausser dem p-Diaminobenzolderivat der Formel (I) zusätzlich mindestens eine weitere Entwicklersubstanz enthält, welche ausgewählt ist aus der Gruppe bestehend aus 1,4-Diaminobenzol, 2,5-Diaminotoluol, 2,5-Diaminophenylethylalkohol, 4-Aminophenol und seinen Derivaten, 4,5-Diaminopyrazolderivaten und Tetraaminopyrimidinen.

9. Mittel nach einem der Ansprüche 5 bis 8, **dadurch gekennzeichnet, dass** die Entwicklersubstanzen und Kupplersubstanzen, bezogen auf die Gesamtmenge des Färbemittels, jeweils in einer Gesamtmenge von 0,005 bis 20 Gewichtsprozent enthalten sind.

10. Mittel nach einem der Ansprüche 5 bis 9, **dadurch gekennzeichnet, dass** es zusätzlich mindestens einen direktziehenden Farbstoff enthält.

11. Mittel nach einem der Ansprüche 5 bis 10, **dadurch gekennzeichnet, daß** es ein Haarfärbemittel ist.

## Claims

1. p-Diaminobenzene derivatives of the general formula (I) or their physiologically compatible, watersoluble salts, 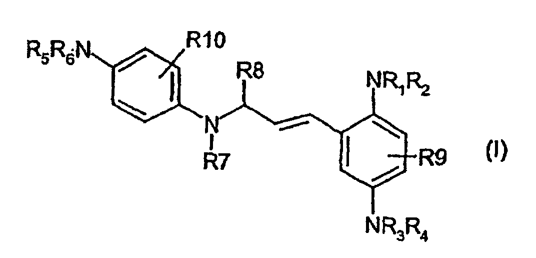 in which
**R1, R2, R3, R4 R5, R6 and R7** independently of one another are hydrogen, a C₁-C₆-alkyl group, a C₁-C₄-hydroxyalkyl group, a C₂-C₄-dihydroxyalkyl group or a C₁-C₄-alkoxy-(C₁-C₄)-alkyl group, or **R1** and **R2** or **R3** and **R4** or **R5** and **R6** form a four-membered to eight-membered aliphatic ring, where at least two of the radicals **R1** to **R7** are hydrogen;
**R8** is hydrogen or a C₁-C₄-alkyl group;
**R9, R10** independently of one another are hydrogen, a hydroxyl group, a halogen atom, a C₁-C₄-alkyl group, a C₁-C₄-hydroxyalkyl group, a C₁-C₄-aminoalkyl group, an amino group, a C₁-C₂-alkylamino group or a C₁-C₄-alkoxy group.

2. p-Diaminobenzene derivative according to Claim 1, **characterized in that**, in the formula (I), (i) at least one of the radicals **R8** to **R10** is hydrogen and/or (ii) **R1** and **R2** or **R3** and **R4** or **R5** and **R6** or all of the radicals **R1** to **R4** or **R1** to **R7** are hydrogen.

3. p-Diaminobenzene derivative according to Claim 1 or 2, **characterized in that**, in the formula (I), **R8** is hydrogen and **R5** and **R6**, independently of one another, are hydrogen, a C₁-C₄-alkyl group, a C₁-C₄-hydroxyalkyl group, a C₂-C₄-dihydroxyalkyl group.

4. p-Diaminobenzene derivative according to one of Claims 1 to 3, **characterized in that** it is chosen from the group consisting of 4-[3-(2,5-diaminophenyl)allylamino]aniline; 4-[3-(2,5-diaminophenyl)allylamino)-2-(2-hydroxyethyl)aniline; 4-[3-(2,5-diaminophenyl)allylamino)-5-(2-hydroxyethyl)aniline; 4-[3-(2,5-diaminophenyl)allylamino)-2-chloroaniline; 4-[3-(2,5-diaminophenyl)allylamino)-2-methylaniline; 4-[3-(2,5-diaminophenyl)allylamino)-3-(2-hydroxyethyl)aniline; 4-[3-(2,5-diaminophenyl)allylamino)-3-chloroaniline; 4-[3-(2,5-diaminophenyl)allylamino)-3-methylaniline; 4-[3-(2,5-diamino-3-(2-hydroxyethyl)phenyl)allylamino)aniline; 4-[3-(2,5-diamino-3-chlorophenyl)allylamino)aniline; 4-[3-(2,5-diamino-3-methylphenyl)allylamino)aniline; 4-[3-(2,5-diamino-6-methylphenyl)allylamino)aniline; 4-[3-(2,5-diamino-4-(2-hydroxyethyl)phenyl)allylamino)aniline; 4-[3-(2,5-diamino-4-chlorophenyl)allylamino)aniline; 4-[3-(2,5-diamino-4-methylphenyl)allylamino)aniline; 4-[3-(N²,N²-bis-methyl-2,5-diaminophenyl) allylamino)-2-aniline; 4-[3-(N²,N²-bis(2-hydroxyethyl)-2,5-diaminophenyl)allylamino)-2-aniline; 4-[3-(N²,N²-bis-methyl-2,5-diaminophenyl)-allylamino)-2-aniline; 4-[3-(N²,N²-bis(2-hydroxyethyl)-2,5-diaminophenyl)allylamino)-2-aniline, 4-[3-(2,5-diaminophenyl)allylamino)-2-N,N'-bis-methyl-aniline; 4-[3-(2,5-diaminophenyl)allylamino)-2-N,N'-bis-(2-hydroxyethyl)aniline or their physiologically compatible salts.

5. Agent for the oxidative colouring of keratin fibres based on a developer substance-coupler substance combination, **characterized in that** it comprises, as developer substance, at least one p-diaminobenzene derivative of the formula (I) according to one of Claims 1 to 4.

6. Agent according to Claim 5, **characterized in that** it comprises the diaminobenzene derivative of the formula (I) in an amount of from 0.005 to 20 per cent by weight.

7. Agent according to Claim 5 or 6, **characterized in that** the coupler substance is chosen from the group consisting of 2,6-diaminopyridine, 2-amino-4-[(2-hydroxyethyl)amino]anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di[(2-hydroxyethyl)amino]-1,5-dimethoxybenzene, 2,3-diamino-6-methoxypyridine, 3-amino-6-methoxy-2-(methylamino)pyridine, 2,6-diamino-3,5-dimethoxypyridine, 3,5-diamino-2,6-dimethoxypyridine, 1,3-diaminobenzene, 2,4-diamino-1-(2-hydroxyethoxy)benzene, 2,4-diamino-1,5-di(2-hydroxyethoxy)benzene, 1-(2-aminoethoxy)-2,4-diaminobenzene, 2-amino-1-(2-hydroxyethoxy)-4-methylaminobenzene, 2,4-diaminophenoxyacetic acid, 3-[di(2-hydroxyethyl)amino]-aniline, 4-amino-2-di[(2-hydroxyethyl)amino]-1-ethoxybenzene, 5-methyl-2-(1-methylethyl)phenol, 3-[(2-hydroxyethyl)amino]aniline, 3-[(2-aminoethyl)amino]aniline, 1,3-di(2,4-diaminophenoxy)propane, di(2,4-diaminophenoxy)methane, 1,3-diamino-2,4-dimethoxybenzene, 2,6-bis-(2-hydroxyethyl)aminotoluene, 4-hydroxyindole, 3-dimethylaminophenol, 3-diethylaminophenol, 5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methylphenol, 3-amino-2-chloro-6-methylphenol, 3-aminophenol, 2-[(3-hydroxyphenyl)-amino]acetamide, 5-[(2-hydroxyethyl)amino]-2-methylphenol, 3-[(2-hydroxyethyl)amino]phenol, 3-[(2-methoxyethyl)amino]phenol, 5-amino-2-ethylphenol, 2-(4-amino-2-hydroxyphenoxy)ethanol, 5-[(3-hydroxypropyl)amino]-2-methylphenol, 3-[(2,3-dihydroxypropyl)amino]-2-methylphenol, 3-[(2-hydroxyethyl)amino]-2-methylphenol, 2-amino-3-hydroxypyridine, 5-amino-4-chloro-2-methylphenol, 1-naphthol, 1,5-dihydroxynaphthalene, 1,7-dihydroxynaphthalene, 2,3-dihydroxynaphthalene, 2,7-dihydroxynaphthalene, 2-methyl-1-naphthol acetate, 1,3-dihydroxybenzene, 1-chloro-2,4-dihydroxybenzene, 2-chloro-1,3-dihydroxybenzene, 1,2-dichloro-3,5-dihydroxy-4-methylbenzene, 1,5-dichloro-2,4-dihydroxybenzene, 1,3-dihydroxy-2-methylbenzene, 3,4-methylenedioxyphenol, 3,4-methylenedioxyaniline, 5-[(2-hydroxyethyl)amino]-1,3-benzodioxole, 6-bromo-1-hydroxy-3,4-methylenedioxybenzene, 3,4-diaminobenzoic acid, 3,4-dihydro-6-hydroxy-1,4(2H)-benzoxazine, 6-amino-3,4-dihydro-1,4(2H)-benzoxazine, 3-methyl-1-phenyl-5-pyrazolone, 5,6-dihydroxyindole, 5,6-dihydroxyindoline, 5-hydroxyindole, 6-hydroxyindole, 7-hydroxyindole and 2,3-indolinedione.

8. Agent according to one of Claims 5 to 7, **characterized in that**, apart from the p-diaminobenzene derivative of the formula (I), it additionally comprises at least one further developer substance which is chosen from the group consisting of 1,4-diaminobenzene, 2,5-diaminotoluene, 2,5-diaminophenylethyl alcohol, 4-aminophenol and its derivatives, 4,5-diaminopyrazole derivatives and tetraaminopyrimidines.

9. Agent according to one of Claims 5 to 8, **characterized in that** the developer substances and coupler substances, based on the total amount of the colorant, are present in each case in a total amount of from 0.005 to 20 per cent by weight.

10. Agent according to one of Claims 5 to 9, **characterized in that** it additionally comprises at least one direct dye.

11. Agent according to one of Claims 5 to 10, **characterized in that** it is a hair colorant.

## Revendications

1. Dérivés de p-diaminobenzène de formule générale (I) ou leurs sels physiologiquement acceptables solubles dans l'eau 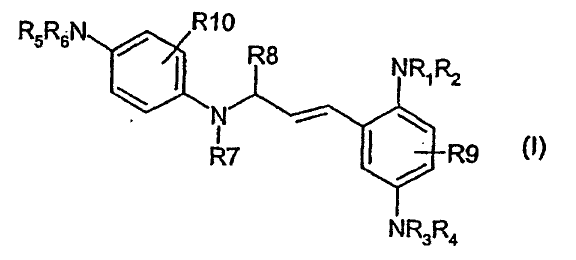 dans laquelle
R1, R2, R3, R4, R5, R6 et R7 signifient indépendamment l'un de l'autre hydrogène, un groupement alkyle en C₁ à C₆, un groupement hydroxyalkyle en C₁ à C₄, un groupement dihydroxyalkyle en C₂ à C₄ ou un groupement (alcoxy en C₁ à C₄) (alkyle en C₁ à C₄) ou R1 et R2 ou, selon le cas, R3 et R4 ou, selon le cas, R5 et R6 forment un cycle aliphatique à quatre jusqu'à huit membres, au moins deux des radicaux R1 à R7 signifiant hydrogène ;
R8 représente hydrogène ou un groupement alkyle en C₁ à C₄;
R9, R10 représentent indépendamment l'un de l'autre hydrogène, un groupement hydroxy, un atome d'halogène, un groupement alkyle en C₁ à C₄, un groupement hydroxyalkyle en C₁ à C₄, un groupement aminoalkyle en C₁ à C₄, un groupement amino, un groupement (alkyle en C₁ à C₂)amine, ou un groupement alcoxy en C₁ à C₄.

2. Dérivé de p-diaminobenzène selon la revendication 1, **caractérisé en ce que** dans la formule (I), (i) au moins un des radicaux R8 à R10 signifie hydrogène et/ou (ii) R1 et R2 ou R3 et R4 ou R5 et R6 ou tous les radicaux R1 à R4 ou selon le cas R1 à R7 signifient hydrogène.

3. Dérivé de p-diaminobenzène selon la revendication 1 ou 2, **caractérisé en ce que** dans la formule (I) R8 représente hydrogène et R5 et R6 représentent indépendamment l'un de l'autre hydrogène, un groupement alkyle en C₁ à C₄, un groupement hydroxyalkyle en C₁ à C₄, un groupement dihydroxyalkyle en C₂ à C₄.

4. Dérivé de p-diaminobenzène selon l'une quelconque des revendications 1 à 3, **caractérisé en ce qu'**il est choisi dans le groupe constitué par la 4-[3-(2,5-diaminophényl)allylamino]aniline ; la 4-[3-(2,5-diaminophényl)allylamino]-2-(2-hydroxyéthyl)aniline ; la 4-[3-(2,5-diaminophényl)allylamino]-5-(2-hydroxyéthyl)aniline ; la 4-[3-(2,5-diaminophényl)allylamino]-2-chloroaniline ; la 4-[3-(2,5-diaminophényl)allylamino]-2-méthylaniline ; la 4-[3-(2,5-diaminophényl)-allylamino]-3-(2-hydroxyéthyl)aniline ; la 4-[3-(2,5-diaminophényl)allylamino]-3-chloro-aniline ; la 4-[3-(2,5-diaminophényl)allylamino]-3-méthylaniline ; la 4-[3-(2,5-diamino-3-(2-hydroxyéthyl)phényl)allylamino]-aniline ; la 4-[3-(2,5-diamino-3-chlorophényl)allylamino]aniline ; la 4-[3-(2,5-diamino-3-méthylphényl)-allylamino]aniline ; la 4-[3-(2,5-diamino-6-méthylphényl)allylamino]aniline ; la 4-[3-(2,5-diamino-4-(2-hydroxyéthyl)phényl)allylamino]aniline ; la 4-[3-(2,5-diamino-4-chlorophényl)allylamino]aniline ; la 4-[3-(2,5-diamino-4-méthylphényl)allylamino]aniline ; la 4-[3-(N²,N²-bisméthyl-2,5-diaminophényl)allylamino]-2-aniline ; la 4-[3-(N²,N²-bis(2-hydroxyéthyl)-2,5-diaminophényl)allylamino]-2-aniline ; la 4-[3-(N⁵,N⁵-bisméthyl-2,5-diaminophényl)-allylamino]-2-aniline ; la 4-[3-(N⁵,N⁵-bis-(2-hydroxyéthyl)-2,5-diaminophényl)allylamino]-2-aniline ; la 4-[3-(2,5-diaminophényl)allylamino]-2-N,N'-bisméthylaniline ; la 4-[3-(2,5-diaminophényl)allylamino]-2-N,N'-bis-(2-hydroxyéthyl)aniline ou leurs sels physiologiquement acceptables.

5. Agent pour la teinture par oxydation de fibres kératiniques à base d'une combinaison de substance de développement et de substance de couplage, **caractérisé en ce qu'**il contient comme substance de développement au moins un dérivé de p-diaminobenzène de formule (I) selon l'une quelconque des revendications 1 à 4.

6. Agent selon la revendication 5, **caractérisé en ce qu'**il contient le dérivé diaminobenzène de formule (I) en une quantité de 0,005 à 20% en poids.

7. Agent selon la revendication 5 ou 6, **caractérisé en ce que** la substance de couplage est choisie dans le groupe constitué par la 2,6-diaminopyridine, le 2-amino-4-[(2-hydroxyéthyl)amino]-anisole, le 2,4-diamino-1-fluoro-5-méthylbenzène, le 2,4-diamino-1-méthoxy-5-méthylbenzène, le 2,4-diamino-1-éthoxy-5-méthylbenzène, le 2,4-diamino-1-(2-hydroxyéthoxy)-5-méthylbenzène, le 2,4-di[(2-hydroxyéthyl)amino]-1,5-diméthoxybenzène, la 2,3-diamino-6-méthoxypyridine, la 3-amino-6-méthoxy-2-(méthylamino)pyridine, la 2,6-diamino-3,5-diméthoxypyridine, la 3,5-diamino-2,6-diméthoxypyridine, le 1,3-diaminobenzène, le 2,4-diamino-1-(2-hydroxyéthoxy)-benzène, le 2,4-diamino-1,5-di(2-hydroxyéthoxy)benzène, le 1-(2-aminoéthoxy)-2,4-diaminobenzène, le 2-amino-1-(2-hydroxyéthoxy)-4-méthylaminobenzène, l'acide 2,4-diaminophénoxyacétique, la 3-[di(2-hydroxyéthyl)amino]aniline, le 4-amino-2-di[(2-hydroxyéthyl)amino]-1-éthoxybenzène, le 5-méthyl-2-(1-méthyléthyl)phénol, la 3-[(2-hydroxyéthyl)-amino]aniline, la 3-[(2-aminoéthyl)amino]aniline, le 1,3-di(2,4-diaminophénoxy)propane, le di(2,4-diaminophénoxy)méthane, le 1,3-diamino-2,4-diméthoxybenzène, le 2,6-bis(2-hydroxyéthyl)aminotoluène, le 4-hydroxyindole, le 3-diméthylaminophénol, le 3-diéthylaminophénol, le 5-amino-2-méthylphénol, le 5-amino-4-fluoro-2-méthylphénol, le 5-amino-4-méthoxy-2-méthylphénol, le 5-amino-4-éthoxy-2-méthylphénol, le 3-amino-2,4-dichlorophénol, le 5-amino-2,4-dichlorophénol, le 3-amino-2-méthylphénol, le 3-amino-2-chloro-6-méthylphénol, le 3-aminophénol, le 2-[(3-hydroxyphényl)amino]acétamide, le 5-[(2-hydroxyéthyl)amino]-2-méthylphénol, le 3-[(2-hydroxyéthyl)amino]phénol, le 3-[(2-méthoxyéthyl)amino]phénol, le 5-amino-2-éthylphénol, le 2-(4-amino-2-hydroxyphénoxy)éthane, le 5-[(3-hydroxypropyl)amino]-2-méthylphénol, le 3-[(2,3-dihydroxypropyl)amino]-2-méthylphénol, le 3-[(2-hydroxyéthyl)amino]-2-méthylphénol, la 2-amino-3-hydroxypyridine, le 5-amino-4-chloro-2-méthylphénol, le 1-naphtol, le 1,5-dihydroxynaphtalène, le 1,7-dihydroxynaphtalène, le 2,3-dihydroxynaphtalène, le 2,7-dihydroxynaphtalène, le 1-naphtolacétate de 2-méthyle, le 1,3-dihydroxybenzène, le 1-chloro-2,4-dihydroxybenzène, le 2-chloro-1,3-dihydroxybenzène, le 1,2-dichloro-3,5-dihydroxy-4-méthylbenzène, le 1,5-dichloro-2,4-dihydroxybenzène, le 1,3-dihydroxy-2-méthylbenzène, le 3,4-méthylènedioxyphénol, la 3,4-méthylènedioxyaniline, le 5-[(2-hydroxyéthyl)amino]-1,3-benzodioxole, le 6-bromo-1-hydroxy-3,4-méthylènedioxybenzène, l'acide 3,4-diaminobenzoïque, la 3,4-dihydro-6-hydroxy-1,4(2H)-benzoxazine, la 6-amino-3,4-dihydro-1,4(2H)-benzoxazine, la 3-méthyl-1-phényl-5-pyrazolone, le 5,6-dihydroxyindole, la 5,6-dihydroxyindoline, le 5-hydroxyindole, le 6-hydroxyindole, le 7-hydroxyindole et la 2,3-indolinedione.

8. Agent selon l'une quelconque des revendications 5 à 7, **caractérisé en ce qu'**il contient, outre le dérivé de p-diaminobenzène de formule (I), en plus au moins une autre substance de développement qui est choisie dans le groupe constitué par le 1,4-diaminobenzène, le 2,5-diaminotoluène, l'alcool 2,5-diaminophényléthylique, le 4-aminophénol et ses dérivés, les dérivés de 4,5-diaminopyrazole et les tétraaminopyrimidines.

9. Agent selon l'une quelconque des revendications 5 à 8, **caractérisé en ce que** les substances de développement et les substances de couplage, par rapport à la quantité totale de la teinture sont à chaque fois contenues en une quantité totale de 0,005 à 20% en poids.

10. Agent selon l'une quelconque des revendications 5 à 9, **caractérisé en ce qu'**il contient en plus au moins un colorant direct.

11. Agent selon l'une quelconque des revendications 5 à 10, **caractérisé en ce qu'**il s'agit d'une teinture pour les cheveux.
